(19) Europäisches Patentamt

European Patent Office

Office européen des brevets

(11) **EP 0 850 069 B1**

(12) **EUROPEAN PATENT SPECIFICATION**

(45) Date of publication and mention
of the grant of the patent:
**03.04.2002 Bulletin 2002/14**

(21) Application number: **95940694.3**

(22) Date of filing: **13.11.1995**

(51) Int Cl.[7]: **A61K 38/00**, A61K 38/04,
A61K 39/145, C07K 1/00,
C07K 2/00, C07K 4/00,
C07K 5/00, C07K 7/00,
C07K 16/00, C07K 17/00,
C12N 15/00

(86) International application number:
**PCT/US95/14698**

(87) International publication number:
**WO 96/14855 (23.05.1996 Gazette 1996/23)**

(54) **METHOD FOR INDUCING HUMORAL AND CELLULAR IMMUNE RESPONSES UTILIZING INTRACELLULAR DELIVERY OF PEPTIDE-COATED MICROPARTICLES**

METHODE ZUR INDUKTION DER HUMORALEN UND ZELLULÄREN IMMUNANTWORT DURCH INTRAZELLULÄRE ZUFUHR VON PEPTIDBESCHICHTETEN MIKROPARTIKELN

PROCEDE DESTINE A INDUIRE DES REPONSES IMMUNITAIRES HUMORALES ET CELLULAIRES PAR APPORT INTRACELLULAIRE DE MICROPARTICULES A ENROBAGE PEPTIDIQUE

(84) Designated Contracting States:
**DE FR GB IT SE**

(30) Priority: **15.11.1994 US 347785**

(43) Date of publication of application:
**01.07.1998 Bulletin 1998/27**

(73) Proprietor: **Powderject Vaccines, Inc.
Madison, Wisconsin 53711 (US)**

(72) Inventors:
• **POHLMANN, Edward C.
Madison, WI 53704 (US)**
• **SHEEHY, Michael J.
Madison, WI 53711 (US)**
• **BARTON, Kenneth A.
Middleton, WI 53562 (US)**

(74) Representative: **Woods, Geoffrey Corlett et al
J.A. KEMP & CO.
Gray's Inn
14 South Square
London WC1R 5JJ (GB)**

(56) References cited:
**US-A- 5 100 792          US-A- 5 120 657
US-A- 5 371 015**

• **AIDS RESEARCH AND HUMAN RETROVIRUSES, Volume 10, Supplement 2, issued 1994, HAYNES et al., "Accell Particle-Mediated DNA Immunization Elicits Humoral, Cytotoxic and Protective Immune Responses", pages S43-S45.**
• **IMMUNOBIOLOGY, Volume 184, issued 1992, NORLEY et al., "Vaccination Against HIV", pages 193-207.**
• **CHEMICAL SOCIETY REVIEWS, Volume 9, issued 1980, BROWN et al., "The Chemistry of the Gold Drugs", pages 218-219.**

**Description**

Field of the Invention

The present invention relates to the field of delivering biological material into cells and more particularly to the induction of immunological response by the delivery of proteins and peptides both intracellularly and extracellularly into animal tissues.

Background of the Invention

Induction of an immune response, whether cytotoxic or humoral, is typically achieved by delivery into the target animal of a quantity of the substance against which the immune response is desired. Small amino acid chains, or protein sub-units, termed peptides, often are incapable of eliciting an immune response when injected into a animal to be immunized. To overcome that limitation, the peptide antigen is often coupled to a proteinaceous carrier or other molecule functioning as a hapten. The carrier-coupled or hapten-coupled peptide is typically introduced intramuscularly or intraperitoneally in the presence of an adjuvant, whereupon both portions of the carrier-peptide complex contribute to the induction of an immune response.

This method can be effective, but suffers shortcomings in that the coupling may alter the immunogenic determinants of the protein such that the immune response generated is not protective against the native pathogen against which protection is sought. Also, while such an immunization produces a humoral (i.e. antibody) immune response, it does not produce a cell-mediated cytotoxic immune response. This same limitation occurs with the injection of whole proteins into animals. Therefore, it has been desired to achieve carrier-free induction of an immune response using a protein or peptide.

Many have tried to direct particular immune responses by introducing short peptide portions of larger proteins as immunogens without the use of an adjuvant. Typically, however, these immunogens have been coupled to "larger protein carriers." While peptides are generally less immunogenic than whole antigenic proteins, shorter peptide fragments may be advantageous when used as vaccines because they may allow certain epitopes to be present without other immunosuppressant epitopes.

In addition to modifying the nature of the peptide, much effort has been directed to identifying adjuvants that are compatible with human therapy. The commonly used experimental adjuvant, Freund's complete adjuvant, while allowed for animal studies, has strong side effects that preclude its use in humans.

Attention is also focused on the use of short peptide fragments of larger proteins as immunogens. The use of synthetic peptides would cut costs and increase the purity of immunogens. However, results have been mixed in generating antibodies that recognize native proteins from synthetic peptide immunogens. Moreover, no one has heretofore successfully devised a method for delivering carrier free synthetic peptides in a manner that results in the induction of a full immune response. The negative results have been blamed on inadequate conjugate stability, alteration of antigenic reactivity after coupling, and selection of antibodies that recognize the peptide-carrier conjugate, but not the peptide alone.

Rousseaux-Prevost et al., Molecular Immunology, 28:943-949 (1991) have demonstrated the production of antibodies from carrier free synthetic peptides of human protamines after injection. However, these authors performed their experiments in the presence of unconjugated bovine serum albumin and did not detect a cytotoxic response.

Tang et al., J. Virol., 62:4745-4751 (1988), have shown that an uncoupled decapeptide was able to elicit specific antibody and T helper cell responses after intraperitoneal injection. It was the understanding of those authors that a certain epitope present on a decapeptide but not on shorter variants of the decapeptide, was effective as a T helper-cell-inducing epitope that functioned in a manner comparable to a T helper cell epitope normally present on a conjugated carrier molecule. While the art had thought it sufficient to couple synthetic B-cell epitopes to carriers containing T helper cell epitopes, that approach has been demonstrated to be undependable in light of reports of carrier-induced epitopic suppression which can modulate B-cell activation.

The induction of cytotoxic T-cells was reported after inoculation with certain influenza hemagglutinin fragments. Sites on such peptides have been identified that are recognized by class-I MHC-restricted cytotoxic T-lymphocytes. Cytotoxic T-lymphocytes are also known to recognize processed antigenic fragments, and this has been shown in the case of fragments of the nucleoprotein of the influenza virus by Townsend et al., Prog. Allergy, 36:10 (1985) and Townsend et al., Cell 39:13 (1984). However, Milich, Advances in Immunology, 45:195 (1989) reports no successful in vivo induction of cytotoxic T-lymphocytes by peptides. Milich laments this shortcoming in the art saying that "the recent findings that peptide fragments can sensitize target cells for lysis by CTLs - and indeed induced CTLs in vitro whereas intact proteins cannot - suggest the possibility that peptide antigens may be used to prime CTL's in vivo if an effective delivery system can be devised." Id at 262.

Therefore, it is apparent that no strategy for delivering protein or carrier-free peptide immunogens will be optimally applied in the area of vaccination until a suitable method for inducing humoral and cellular immune responses is developed that is effective, affordable and safe.

Summary of the Invention

The present invention is summarised in that humoral and cellular immune responses can be induced after direct 'delivery into target cells of peptides or proteins immobilized on biologically inert, non-biological particles.

It is an object of the present invention to provide a method for inducing both a humoral and a cell-mediated immune response in an organism by direct introduction of biological carrier-free peptides or proteins into cells.

Accordingly, the present invention provides use of an immunogen in the manufacture of a particulate medicament for accelerating into a target cell of a mammalian subject in an amount sufficient to induce in the mammal an immune response specific to the said immunogen, wherein the immunogen is coated on a biologically inert particle having sufficient density to be delivered directly into the target cell.

The present invention also provides:

- use of an immunogenic peptide or protein in the manufacture of a particulate medicament for human vaccination, wherein:

    (a) the immunogenic peptide or protein is coated on biologically inert particles,
    (b) the said vaccination is performed by accelerating the coated particles into target skin cells of a human, and
    (c) the biologically inert particles have a size and density sufficient to be delivered directly into the target cells.

It is thus a feature of the present invention that antigenic determinants of a peptide can be retained after direct intracellular delivery of the peptide into target cells.

It is an advantage of the present invention that delivery of the immunogen is rapid and efficient. It is another advantage of the present invention that it appears equally applicable to peptides of all sizes ranging from very small peptides to large proteins.

Brief Description of the Drawings

    Fig. 1 is a schematic depiction of an apparatus that can be used in connection with the present method.
    Fig. 2 is a graphical representation of some of the results of Example 5.
    Fig. 3 is a graphical representation of some of the results of Example 5.
    Fig. 4 is a graphical representation of some of the results of Example 5.

Detailed Description of the Invention

In the present invention, copies of one or more immunogenic peptides or proteins are attached to a plurality of biologically inert carrier particles. The particles bearing the immunogenic peptides or proteins are delivered into a plurality of skin cells of a target animal whereupon a measurable immune response is induced. No carrier protein or adjuvant is required and the peptide delivery elicits both a humoral and a cytotoxic immune response in the treated animal.

In this application, an immunogenic peptide is intended to encompass any biological molecule that has as all or part of its structure an amino acid chain of sufficient length and suitable sequence to induce an immune response in accord with the present invention. This is specifically intended to encompass molecules generally known as peptides, polypeptides, and proteins as well as molecules that include peptide, polypeptide or protein portions in their structure, such as glycopeptides. Although the terms are used herein to have differing meaning, i.e. peptides are sub-units of proteins, all such molecules are intended to fall within the scope of the present invention.

The immunogenic peptide may range in length from a few amino acids to a full length protein or complex of a protein or proteins. The peptide may have less than 15 amino acid residues. It is also envisioned that the method may be used with complex mixtures or structures that contain antigenic peptides or proteins such as cellular antigens from pathogens or viral capsids or coat protein antigens. The immunogen may be a whole, inactivated virus. The peptide must be of sufficient length to include at least one epitope or determinant that can be recognized as foreign in the target animal's immune system and can induce either a B-cell or a T-cell response. T-cell responses can include inducing cytotoxic T lymphocytes (CTL) or T helper cells, or other T-cell subpopulation. It is intended that peptide sequences used can include a plurality of determinants directed to T- or B-cell activation, or both. As the length of the peptide increases and the number of determinants increase as well, analysis of the induced immune response can become increasingly complex. Of course, the number of determinants on a particular polypeptide will vary with its primary, secondary and tertiary structures and with its length.

Peptides introduced in this method induce a cellular as well as an antibody immune response As is shown in the Examples below, a peptide of nine amino acids in length is adequately long to induce both B- and T-cell responses in this method.

The immunogenic peptide can be a naturally occurring peptide, mimic a peptide found in nature, or have unique features lacking in nature. For example, it could be a non-naturally occurring peptide produced in vitro using standard genetic engineering practices, a synthetic peptide or a cleavage product of any such peptide. A synthetic peptide can be synthesized chemically from individual amino acids or from a plurality of shorter peptides.

The choice of immunogenic peptide can be made by reference to existing information about determinants of known peptides or can be predicted from structural analysis of primary amino acid sequences. The art is cognizant of various methods for predicting the three dimensional structure of a peptide on the basis of known properties of component amino acids. From a hypothesized three dimensional structure, one can make informed predictions about the immunogenicity of particular portions of a peptide molecule.

The method is believed to be operative for any peptide sequence, without regard to its origin. The peptide sequence can derive, for example, from viral, fungal, animal, plant, or microbial protein, since the processes involved in producing an immune response within an animal do not depend upon the source of immunogen.

The immunogenic peptide is attached to particles that are sufficiently small that they can be delivered directly through-cell membranes without lasting damage. The art is generally cognizant of the desirable properties of particles used for particle acceleration into cells. The particles should also have sufficient density to acquire adequate momentum during acceleration to be able to pass directly into cells. High density'metal particles of 0.5-5 microns in size are preferred. Most preferred are amorphous gold particles of 0.5-3 microns in size, referred to hereinafter'as gold powder. Gold spherical particles may also be satisfactorily used. Other particles that can be used in a particle acceleration apparatus are known to the art.

The peptide molecules can be attached to the carrier particles either by simply mixing the two in an empirically determined desirable ratio or by chemically coupling-the peptide-to the carrier-particles. The immunogen can thus be chemically bound to the particles. Chemical coupling of a peptide to carrier particles can allow the coupled peptide to present an antigenic site having a desired tertiary structure. The coupling of L-cysteine residues to gold (0), (1), and (III) is described by Brown and Smith, "The Chemistry of the Gold Drugs," Chemical Society Reviews 9:271-311 (1980).

In a preferred method, the peptide is dissolved in absolute ethanol or water or an alcohol/water mixture and a known quantity of the peptide is added to a known quantity of the carrier particles. The mixture is dried under a stream of air or nitrogen gas while vortexing. Alternatively, the peptide can be dried onto the carrier particles by centrifugation under vacuum or precipitated onto the carrier particles. Once dried, large clumps of gold particles coated with peptides form. The dried carrier particles coated with peptide are re-suspended in ethyl acetate. Resuspension is aided by breaking the clumps with a pipette tip followed by water bath sonication until the suspension is uniform.

A desired amount of the uniform peptide-coated carrier particles are then prepared for delivery into target skin cells. It is generally convenient that the peptide be prepared for delivery in a pre-measured form, such as fixed in a form appropriate for the desired dosage, or some fraction thereof. The amount of particles needed to induce cytotoxic and humoral response upon delivery can depend upon the loading rate of the peptide on the particles. It may be necessary to adjust the absolute amounts of material delivered into the target cells to account for differences in peptide length. For any given peptide, titrations of varying amounts of peptide per particle and varying numbers of particles per delivery are recommended. It has been determined empirically that two doses of about 5 µg of a nine amino acid influenza peptide can be sufficient to induce the desired immune response. It is likely that smaller total dosages, as low as 1 or 2 µg, or higher dosages, up to 100 µg, would also work. The experience of the inventors indicates that there is an optimal loading rate, below which the immune response is reduced and above which no linear increase in response is observed. It is understood that the induction of immune responses will likely vary somewhat with the immunogenicity of the polypeptide used and possibly with the peptide molecular weight. The need for optimization of the delivered protein dosage, like that of any pharmaceutical, is to be expected.

Various particle acceleration devices are known and the choice of one over another is not critical. The current device designs employ an explosive, electric or gaseous discharge to propel the coated carrier particles toward the target cells. The coated carrier particles can themselves be releasably attached to a movable carrier sheet or can be removably attached to a surface along which a gas stream passes, lifting the particles from the surface toward the target. An example of a gaseous discharge device is described in U.S. patent 5,204,253. An explosive-type device is described in U.S. Patent 4,945,050. One example of an electric discharge-type particle acceleration apparatus is the ACCELL instrument manufactured by Agracetus, Middleton, Wisconsin which is described in U.S. Patent Number 5,120,657. Another electric discharge apparatus is described in U.S. Patent Number 5,149,655. All of these patents are incorporated herein by reference.

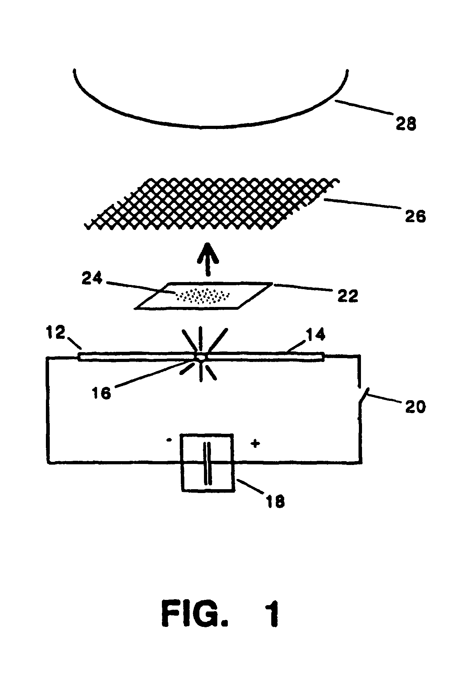

In this work, an electric discharge apparatus like that of US Patent 5,149,655 was used and a gaseous discharge device was also used. Briefly, the apparatus, shown schematically in Fig. 1, works as follows.

As shown in Fig. 1, a pair of electrodes 12 and 14 are provided spaced apart with a spark gap distance between them. The spark gap distance is bridged by a drop of water 16. The end of each of the electrodes 12 and 14 is connected to one terminal of a high voltage capacitor 18, with one of the terminals being connected through a switch 20. After the capacitor 18 is charged and when the switch 20 is closed, high voltage electrical energy is transferred from the capacitor 18 to create a potential between the electrodes 12 and 14. If the potential is sufficiently high, in the order of several kilovolts, a spark will bridge the gap between the electrodes 12 and 14. The electrical spark bridging the electrodes 12 and 14 instantly vaporizes the water vapor droplets 16. The expanding shock wave created by the instant vaporization of the water droplet 16 propagates radially outward in all directions. Previously placed within the zone which is affected by the shockwave is a carrier sheet 22. The carrier sheet 22 has previously been coated with a number of tiny carrier particles 24. The carrier particles 24 are of very dense material, preferably an inert metal such as gold, and are of an extremely small size, on the order of a fraction of a micron to a few microns in size. The carrier particles 24 are of dense material so that they will readily retain momentum and are sufficiently small sized so that they are small in relation to the cells of the organism which they are intended to immunize. It has been found that carrier particles of a size of a few microns can enter living cells, by penetrating the cell walls thereof, without unduly adversely affecting the ability of most of the living cells to survive. In other words, the carrier particles can enter living cells without killing them. The carrier particles in an apparatus such as that shown in Fig. 1 are also coated. The carrier particles 24 are coated with a peptide, or protein, which is intended to be inserted into the living cells of the target organism.

In the general scheme of the apparatus of Fig. 1, the carrier sheet 22 is propelled upward by the expanding shock wave from the vaporization of the water droplets 16. The carrier sheet travels upward until it impacts a retaining screen 26 which, as its name implies, is simply a rigid metallic screen intended to retain the carrier sheet 22. When the carrier sheet 22 hits the retaining screen 26, it stops. However, the momentum of the carrier particles 24 carries them upward from the retaining sheet 26 into the target organism 28. The target organism is the biological organism, tissue or cell culture into which it is desired to transfer the genetic material coated onto the carrier particles 24.

When delivering peptide-coated carrier particles into animal skin, it is desirable to remove hair from the target area. This is readily accomplished by shaving or dipping the target skin area. After depilatory treatment, the target areas are thoroughly rinsed with water.

The force needed to deliver peptide-coated particles into skin cells depends upon the particular target animal and upon the selected target site. Forces comparable to those used in the art to deliver DNA-coated carrier particles into skin cells are appropriate for use in the present method. For example, when using an electric discharge-type apparatus, delivery into skin cells is accomplished at discharge voltages in the range of 15 kV to 18 kV for mice and for primates. One of ordinary skill in the art will recognize that through routine optimization, appropriate delivery forces for delivery into human skin can be determined. It is likely that the force necessary to deliver protein-coated particles into human skin will approximate the force needed for delivery into primates.

Using this method, an immune response can be induced in a mammalian animal having a functional immune system. The method will find particular applicability as a method for vaccinating humans. Because of the many similarities between the immune systems of humans and rodents, and the convenience of rodents, particularly mice, as laboratory animals, rodents are a second desirable target animal as a model for human treatment. It is also envisioned that the present method will be useful in the production in experimental animals of antisera and of antibody-producing B-cell clones having desired specificity for generating hybridomas.

In the examples below, the efficacy of the method of the present invention is demonstrated by comparison in side-be-side tests with peptides delivered in Freund's adjuvant into test animals. The results demonstrate that similar levels of antibody can be achieved using the accelerated particle delivery method as compared to injection with Freund's adjuvant, but injection with Freund's fails to engender the cytotoxic response that delivery by the present invention engenders. Since delivery with Freund's is unsuitable for use with humans, the method of the present invention offers a methodology that is not only superior to the standard method but is also more suitable for transition from animal models to human applications using similar protocols.

Delivery of an amount of the peptide effective to induce a humoral or a cytotoxic response in the target mammalian animal may be achieved by priming the animal with an initial amount of peptide and then boosting the animal at least once according to an empirically determined regimen. For some antigens, as the HIV gp 120 peptide, it has been found that a single dose, i.e. the prime, is all that is required to induce a cytotoxic cellular response. A regimen effective to induce immune responses against a particular influenza antigen is also shown in the Examples. It is not believed that there is anything so distinctive about the tested peptides that would prevent generalizing the method to other immunogenic peptides.

One of ordinary skill would be readily able to formulate a regimen appropriate for another immunogenic peptide using standard protocol-design methods. Variables one would need to consider in devising a delivery regimen would include the size and antigenicity of the peptide, or combination of peptides, the amount of peptide per delivery, the time between priming and boosting and the period of latency preceding the appearance of immune responses. Each of these variables could have an impact, separately or in combination, upon the extent of the immune response induced. Presumably, by carefully adjusting the delivery regimen to take these variables into account, one can optimize the process to achieve a suitably strong immune response in an acceptably short time period.

In designing a regimen for human vaccination, preliminary design efforts could be performed in a closely related model species having an immune system comparable in sophistication to the human immune system. Suitable model mammalian species could be selected from the rodent or primate orders.

After delivery, induction of humoral and cellular immune responses are monitored using known techniques. For example, antibody concentration is readily determined using standard enzyme immunoassays. T-lymphocyte cytotoxicity may be monitored in experimental animals and in humans using standard CTL chromium-release or other assays. Other methods for monitoring the specific immune response may also be used.

Although it was hoped that the intracellular delivery of peptides would facilitate the development of a CTL response, the precise nature of the induction of humoral and cytotoxic immune responses by the disclosed method is not, at present, known. However, it is clear from the accompanying experimental data that both responses are induced by the method without the use of carrier protein or adjuvant.

Furthermore, while it is an advantage of this method that both cellular and humoral immune responses can be induced, success and desirable results can be achieved in certain cases by inducing either response but not both. Therefore, the invention is not to be limited to cases where both responses are induced.

The present invention will be more fully understood by reference to the following examples, which are intended to be merely exemplary of the invention. In the examples, mice have been used as a model recipient for an immunogenic peptide. Mice and other rodents are standard models for human immune responses.

EXAMPLES

1. Preparation of Peptide Samples for Delivery.

A peptide having the amino acid sequence of TYQRTRALV that resembles a portion of the influenza nucleoprotein (NP) was purchased from the Peptide Laboratory, Inc. (Berkeley, CA). This peptide sequence is presented as SEQ ID NO: 1.

The peptide was suspended at a concentration of 10 µg/µl of 100% ethanol. 25 µl of this peptide solution was mixed with 10 mg of gold powder of 1-3 µ diameter. The mixture of gold powder and peptide was dried under a stream of nitrogen gas. The dried beads, now coated with the peptide, were resuspended in ethyl acetate to a total volume of 3 ml. To thoroughly and evenly resuspend the coated gold, large clumps were broken up with a pipette tip and then the suspension was sonicated in a water bath at 16°C until the particles were uniformly suspended. Finally, 163 µl of this gold/peptide suspension was layered gently onto 1.8 cm by 1.8 cm Mylar sheets. After the particles settled from the suspension, the meniscus was broken and the Mylar sheet was air dried.

An unrelated peptide having the amino acid sequence of $NH_2$-Arg-Ile-Gln-Arg-Gly-Pro-Gly-Arg-Ala-Phe-Val-Thr-Ile-Gly-Lys, originally obtained from the HIVgp120 protein, was also coated onto gold powder in the same manner as was described above. To determine the extent of peptide loading onto the gold, a tritiated ($H^3$) version of the HIV peptide was carried through the loading procedure, and the number of bound counts was determined. From this analysis, it was determined that each carrier sheet contained approximately 5 µg of peptide. The tritiated peptide assay indicated that only about one-third of the amount of the peptide added to the gold beads ultimately remained bound to the carrier sheet. It was assumed that the loading rate of the influenza NP peptide onto the gold particles was comparable to that of the HIVgp120 peptide.

2. Sample delivery.

BALB/c mice were anesthetized with 350 µl of Ketamine/Xylazine solution (5 g/l Ketamine, 1.2 g/l Xylazine). Abdominal target areas were shaved, then were treated with depilatory (Nair, Carter-Wallace, NY) for 2 minutes to remove residual stubble and the stratum corneum, and rinsed with water.

The coated Mylar sheets described in the previous section were loaded into an ACCELL particle acceleration instrument (Agracetus, Middleton, Wisconsin) for delivery. At each priming and boost inoculation, each mouse was treated with two coated sheets for a total of approximately 10 µg of peptide per treatment. Mice in this study were treated with gold powder either coated with the influenza NP peptide or coated with the HIVgp120 peptide.

A separate control procedure using standard immunization protocols was also carried out in the same experiment. In the standard inoculation procedure, the peptide was mixed with Freund's Complete Adjuvant (FCA). 3.0 ml of a 200 µg/ml solution of either the influenza or HIV peptide in 100% ethanol were added to 1.5 ml of phosphate buffered saline and 1.5 ml of FCA. BALB/c mice were each inoculated with 100 µl of this mixture on day 0.

Before inoculation, a serum sample was taken from each mouse. A portion of each group of mice were boosted using the same procedure (injection or particle-mediated peptide delivery respectively), at 28 days and at 42 days post inoculation. Mice being studied in the control immunization protocol were boosted with 100 μl of the appropriate peptide with Freund's Incomplete Adjuvant, in the same relative concentrations as were described in connection with the original inoculation. Thus all mice received approximately 10 μg of peptide per immunization.

The generation of an anti-NP peptide-specific immune response was monitored at 14 days, 42 days and 56 days after initial inoculation. On each of these days, three mice of each group were tested for production of NP peptide-specific cytotoxic T-lymphocytes (CTL) and also for antibody production. In each experiment, at least one naive (uninoculated) mouse was also tested.

3. Test for CTL.

The cytotoxic T-lymphocyte protocol was a standard assay using mouse spleen cells. Mice were sacrificed by $CO_2$ inhalation and their spleens were removed aseptically. The spleens were placed in sterile 60 mm petri dishes with 3.0 ml of RPMI 1640 culture medium plus 10% heat inactivated fetal bovine serum and 0.05 mg/ml gentamycin. The spleens were minced into fine pieces using sterile scissors and were extruded through a 70 micron pore size nylon mesh cup (Falcon) into a 50 ml sterile polypropylene tube. The petri dish and the nylon mesh cup were washed with 10.0 ml of the RPMI 1640 plus 10% FBS plus gentamycin medium. The splenocytes were centrifuged at 1500 RPM for 5 minutes and the supernatant was discarded. The pellet was resuspended in sterile Ack lysis buffer (0.15 M $NH_4Cl$, 1.0 mM $KHCO_3$, 0.1 mM $Na_2EDTA$, pH 7.2-7.4) to remove red blood cells using approximately 5 ml per spleen. The spleen cells in Ack lysis buffer were incubated at ambient temperature for 5 minutes with occasional shaking. Heat inactivated RPMI plus 10% FBS was added to fill the tube and it was spun for 10 minutes at 300 x g to pellet the cells. The pellet was resuspended in 10 ml of the RPMI medium. Approximately $1.5 \times 10^8$ splenocytes were obtained from each spleen.

Stimulator splenocytes from untreated mice were used to activate the responder splenocytes obtained from immunized mice in sensitization medium (SM). SM was made from RPMI 1640 plus 10% FBS plus 0.05 mg/ml gentamycin, 1 mM sodium pyruvate, nonessential amino acids, and 10 units/ml rat IL-2.

Stimulator splenocytes were prepared as follows. The cells at $1 \times 10^7$ per ml were exposed to mitomycin C at 25 μg/ml for 20 minutes at 37°C in a 5% $CO_2$ atmosphere in the dark. The cells were resuspended and were washed 3 times with 50 ml of the RPMI medium. After each wash, the cells were centrifuged at 300 x g for 5 minutes. The peptide was dissolved at a concentration of 1.0 mg per 100 μl of DMSO and then 10 μl of this solution was added to 10 ml of SM medium, making the final peptide concentration approximately $1 \times 10^{-5}$ molar for both peptides.

The stimulator and responder splenocytes were co-incubated in T25 flasks for 6 days at 37°C in a 5% $CO_2$ atmosphere in 10 ml of SM.

A chromium release assay was employed to measure the ability of in vitro-stimulated responder cells to lyse peptide-pulsed P815 cells (TIB64, ATCC). Effector cells were harvested from the culture flasks and put into 96-well round-bottom culture plates at varying numbers of viable cells per well (to achieve varying effector:target cell ratios). The P815 cells were labeled with $^{51}Cr$ by adding 0.1 mCi of radiolabeled sodium chromate (NEN) in 0.2 ml of normal saline to an aliquot of cells in 0.1 ml of the RPMI medium. The P815 target cells were incubated with the $^{51}Cr$ for 1 hour at 37°C and then washed three times with the RPMI medium. The cells were pelleted and re-suspended at a concentration of $1 \times 10^4$/ml in the RPMI medium with the influenza NP peptide at $1 \times 10^{-5}$ M. The target cells were peptide pulsed for 1 hour at 37°C. Without further washing, radiolabeled, peptide-pulsed target cells were added to the effector cells in the 96 well culture plate at $1 \times 10^4$ cells per well in 0.1 ml of the RPMI medium. After 4 hours incubation at 37°C, the 96-well plates were centrifuged and 0.1 ml of each cell supernatant was subjected to liquid scintillation counting to measure lysis induced by the responder cells. The percent of specific lysis of labelled target cells for a particular effector cell sample was calculated as follows:

$$\frac{(^{51}Cr \text{ release in sample} - {}^{51}Cr \text{ release in control}) \times 100\%}{(\text{Maximum } {}^{51}Cr \text{ release} - \text{control } {}^{51}Cr \text{ release})}$$

The control $^{51}Cr$ release is the amount of radioactivity released from target cells that receive no effector cells. The maximum chromium release is the amount of radioactivity released after complete lysis of target cells brought about by the addition of 1% Triton X-100. The following results were obtained. The lack of response of cells from the gp 120-treated mice demonstrates the specificity of the induction.

TABLE 1

| AVERAGE % SPECIFIC LYSIS OF TARGET CELLS PULSED WITH INFLUENZA NP PEPTIDE AT EFFECTOR/ TARGET RATIO OF 50:1[A] | | | | |
|---|---|---|---|---|
| Peptide Used for Immunization | Immunization Method | Day 14 | Day 42 | Day 56 |
| Influenza NP | accelerated particle | 14.3 | 24.3 | 0.6 |
| | | 12.7 | 38.1 | 2.9 |
| | | 28.0 | 9.9 | 1.8 |
| | conventional injection | -3.5 | 1.1 | -1.7 |
| | | -3.6 | -0.6 | -1.7 |
| | | -9.1 | 1.3 | |
| HIV gp 120 | accelerated particle | -2.5 | -2.7 | -1.9 |
| | | | -1.1 | -1.6 |
| | | | -1.7 | -0.9 |
| | conv. injection | -1.5 | -0.6 | -2.1 |
| | | | -1.4 | -0.6 |
| | | | -1.7 | -1.7 |
| No treatment | | -4.7 | -1.6 | -1.9 |
| | | 1.3 | -1.3 | -1.8 |

[A]Each number represents the average percent specific lysis for a single mouse.

4. Antibody Production.

The blood samples collected from each mouse before inoculation and on days 14, 42, and 56 were centrifuged at 2000 x g for 5 minutes in serum separator tubes (Microtainer tubes). Serum samples were stored at-20°C before analysis. The wells of 96-well high-binding EIA/RIA plates (Costar) were coated with influenza viral particles that had previously been lysed with extraction buffer (0.5 M Tris-HCl, pH 7.5-7.8, 0.6 M KCl and 0.5% Triton X100). The virus particles were lysed at a concentration of 0.5 ml of extraction buffer per 20,000 hemagglutination (HA) units. After 5 minutes at ambient temperature, the extraction buffer was diluted with PBS to 5.0 ml. Each well received 50 µl of viral extract that contained 200 HA units. After overnight incubation, the supernatant, containing any unbound viral antigen, was aspirated and the plates were washed 3 times with PBS plus 0.025% Tween-20 (BioRad). The wells were then blocked with 300 µl per well of 2% skim milk powder (Carnation) in PBS for 1 hour at 37°C, and then washed 3 times with PBS/Tween-20. Serum samples were diluted serially in PBS/Tween-20. Aliquots (50 µl) of serially diluted serum samples were added to individual wells and plates were incubated overnight. The plates were then washed 3 times with PBS/Tween-20. A 1:1500 dilution of a goat anti-mouse IgG (H+L) alkaline phosphatase conjugate (BioRad) in PBS/Tween-20 was added at 50 µl per well and plates were incubated for 1 hour at 37°C. The wells were washed 3 times with PBS/Tween-20. Alkaline phosphatase substrate BioRad (100 µl) was added and plates incubated for 30 minutes at room temperature. Color development was stopped by adding 100 µl of 0.4 normal NaOH and the plates were read at 405 nm. Tables 2 and 3 demonstrate that antibody production after delivery of biologically active peptides by particle acceleration is similar to the antibody production after classical inoculation using Freund's adjuvant.

5. Protection From Lethal Virus Challenge.

Gold particles coated with killed influenza virus were used in an attempt to immunize mice against a lethal flu virus challenge. A killed virus preparation was made by lysing flu virus strain A/PR/8/34 with the same solution of Tris-HCl,KCl and 0.5% Triton X100 solution as described in example 4. Viral proteins corresponding to approximately 14,500 HA units were speed vacuum dried onto 10 mg of 0.95 µ diameter gold particles. Acetone (1.0 ml) was added, and tubes were sonicated and vortexed to break up aggregates.

To prepare cartridges for use with the gaseous discharge particle acceleration device, the slurry of protein-coated-gold in acetone was aspirated into a horizontal length of Teflon tubing. After 5 minutes, the virus-coated gold particles had settled, and the acetone was removed. The tubing was then dried with a gentle stream of $N_2$ gas. For assay and peptide delivery, the tubing was cut into (1/2") 1/2 x 2·54 cm lengths. Three (1/2") 1/2 x 2·54 cm lengths were assayed by the Bradford protein assay to correlate HA units with absorbance units, and the three tubes were determined to have 256, 492, and 496 HA units, respectively.

The remaining (1/2") 1/2 x 2·54 cm lengths of tubing were used to immunize the mice. A gene delivery device based on a compressed gas passing through the tubing was used. Six mice were primed on day 0 by delivery of killed-viruscoated gold particles at two abdominal sites and boosted on day 31 by a similar treatment at three abdominal sites. On day 40, blood samples were collected for antibody determination. One mouse had an anti-viral antibody titer of 1/500 by ELISA (Same assay described in example 4), and the remaining five mice had ELISA titers of 1/5,000 to 1/20,000. Six mice were left untreated, to serve as non-immunized controls.

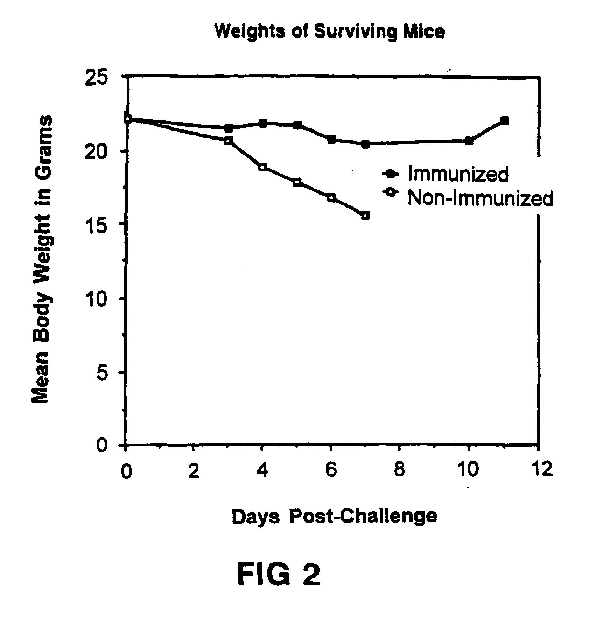

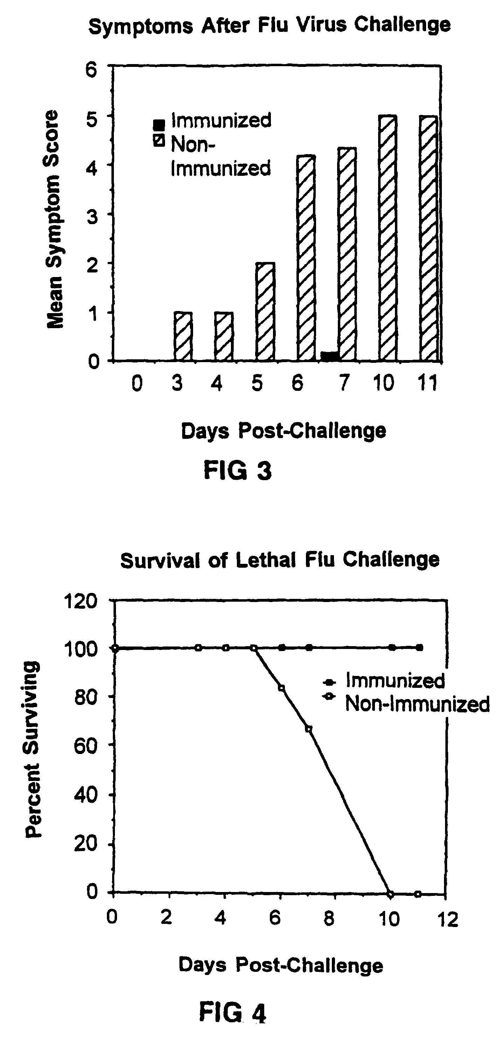

On day 62 following the initial immunization, the six immunized-and the six non-immunized mice-were all challenged with a normally lethal dose (400 plaque-forming units)' of the same virus strain used for' the vaccinations. As shown in Figures 2, 3 and 4; the six non-immunized mice all developed severe-symptoms and died within 10 days; only 1 of 6 mice vaccinated with the protein/gold preparation had temporary weight loss and mild symptoms, while the remaining 5 vaccinated mice remained asymptomatic throughout the experiment. Fig. 2 shows weight loss following virus challenge. Only 1/6 immunized mice temporarily lost weight, whereas 6/6 non-immunized mice lost at least 30% of their weight. No mice remained alive by day 10 post-challenge. Fig. 3 shows symptom scores following virus challenge. A score of 0 indicates no symptoms, while 5 denotes death. Intermediate symptoms were rated as +1 (least severe) to +4 (most severe). Only 1 mouse in the vaccinated group temporarily had symptoms at the mildest (+1) level. Fig. 4 shows the percent of mice surviving at various times after virus challenge.

This protection was most likely due to the delivery of viral proteins to the vaccinated mice, although a contribution of the virus' RNA genome is theoretically possible. RNA is generally unstable, and no attempt was made to protect it from degradation. Moreover, the vaccinated mice did not become ill from the vaccination, suggesting that the RNA genome was not intact. Nevertheless, additional experiments are needed to rule out a possible contribution of the viral RNA to the protective effect observed.

Taken together, the data accumulated in these examples indicate that while the classical inoculation protocol is adequate to induce an antibody response, no comparable cytotoxic cell-mediated immune response arises. In contrast, delivery of biologically active peptides by particle acceleration can induce both humoral and cell-mediated immune responses. Given that inoculation by accelerated particles is very rapid and easy, does not require adjuvants, and that it offers advantages for long term storage of samples, these results demonstrate the desirability of inducing both humoral and cytotoxic immune responses in mammalian animals by particle acceleration, and suggest the ability to deliver effective peptide vaccinations and a range of immunogenic agents using this technology.

TABLE 2

| Antibody Responses to 9-Amino Acid Flu Peptide | | |
| --- | --- | --- |
| | Accell® (No Adjuvant) | Injection with Freund's Adjuvant |
| Prime only | 0/9* | 1/8*(400)** |
| Prime + 1 boost | 1/6(200) | 1/5(200) |
| Prime + 2 boosts | 3/3(200,800,200) | 1/2(400) |

\* Number of mice with detectable antibody/total number of mice tested.

\*\*Reciprocal titers for mice having detectable antibodies.

Table 3

| Antibody Responses to Human Alpha 1 Anti-Trypsin Protein | | |
| --- | --- | --- |
| | Accell® (No Adjuvant) | Injection with Freund's Adjuvant |
| Prime only | 4/4(350,375, 210,250) | 4/4(1425,24000, 1425, 400) |
| Prime + 1 boost | 4/4(9500,9500, 6500,5500) | 4/4(10000,50000, 18000,3800) |

**Claims**

1. Use of an immunogen in the manufacture of a particulate medicament for accelerating into a target cell of a mammalian subject in an amount sufficient to induce in the mammal an immune response specific to the said immunogen, wherein the immunogen is coated on a biologically inert particle having sufficient density to be delivered directly into the target cell.

2. Use according claim 1, wherein the immune response is **characterized by** a cellular response specific for the said immunogen.

3. Use according to claim 1, wherein the immune response is **characterized by** an antibody response specific for the said immunogen.

4. Use according to claim 1, wherein the immune response is **characterized by** both a cellular and an antibody response specific for the said immunogen.

5. Use according to any one of the preceding claims, wherein the coated particle is accelerated into the cell by the force generated by a gaseous discharge.

6. Use according to any one of claims 1 to 4, wherein the coated particle is accelerated into the cell by the force generated by an electric discharge.

7. Use according to any one of the preceding claims, wherein the immunogen is chemically bound to the particle.

8. Use according to any one of the preceding claims, wherein the immunogen is a peptide.

9. Use according to claim 8, wherein the peptide has less than 15 amino acid residues.

10. Use according to any one of claims 1 to 7, wherein the immunogen is polypeptide.

11. Use according to any one of claims 1 to 7, wherein the immunogen is a glycopeptide.

12. Use according to any one of claims I to 7, wherein the immunogen is a whole, inactivated virus.

13. Use according to any one of the preceding claims, wherein the target cell is a skin cell.

14. Use according to any one of the preceding claims, wherein the mammalian subject is a human.

15. Use according to any one of the preceding claims, wherein each particle is a metal particle of 0.5 to 5 $\mu$m in size.

16. Use according to claim 15, wherein the particle is an amorphous gold particle of 0.5 to 3 $\mu$m in size.

17. Use of an immunogenic peptide or protein in the manufacture of a particulate medicament for human vaccination, wherein:

    - the immunogenic peptide or protein is coated on biologically inert particles,
    - the said vaccination is performed by accelerating the coated particles into target skin cells of a human, and
    - the biologically inert particles have a size and density sufficient to be delivered directly into the target cells.

**EP 0 850 069 B1**

**Patentansprüche**

1. Verwendung eines Immunogens bei der Herstellung eines aus Partikeln bestehenden Medikaments zur Akzelerierung in eine Zielzelle eines Säugers hinein in ausreichender Menge, um im Säuger eine für dieses Immunogen spezifische Immunantwort hervorzurufen, wobei das Immunogen auf einen biologisch inerten Partikel von ausreichender Dichte aufgeschichtet wird, um direkt in die Zielzelle beförderbar zu sein.

2. Anwendung nach Anspruch 1, wobei die Immunantwort durch eine für dieses Immunogen spezifische zelluläre Reaktion gekennzeichnet ist.

3. Anwendung nach Anspruch 1, wobei die Immunantwort durch eine für dieses Immunogen spezifische Antikörper-Reaktion gekennzeichnet ist.

4. Anwendung nach Anspruch 1, wobei die Immunantwort sowohl durch eine für dieses Immunogen spezifische zelluläre Reaktion als auch Antikörper-Reaktion gekennzeichnet ist.

5. Anwendung nach einem der vorangehenden Ansprüche, wobei der beschichtete Partikel in die Zelle durch die durch eine Gasentladung erzeugte Kraft akzeleriert wird.

6. Anwendung nach einem der Ansprüche 1 bis 4, wobei der beschichtete Partikel in die Zelle durch die durch eine elektrische Entladung erzeugte Kraft akzeleriert wird.

7. Anwendung nach einem der vorangehenden Ansprüche, wobei das Immunogen chemisch an den Partikel gebunden ist.

8. Anwendung nach einem der vorangehenden Ansprüche, wobei das Immunogen ein Peptid ist.

9. Anwendung nach Anspruch 8, wobei das Peptid weniger als 15 Aminosäure-Reste aufweist.

10. Anwendung nach einem der Ansprüche 1 bis 7, wobei das Immunogen ein Polypeptid ist.

11. Anwendung nach einem der Ansprüche 1 bis 7, wobei das Immunogen ein Glykopeptid ist.

12. Anwendung nach einem der Ansprüche 1 bis 7, wobei das Immunogen ein vollständiges, inaktiviertes Virus ist.

13. Anwendung nach einem der vorangehenden Ansprüche, wobei die Zielzelle eine Hautzelle ist.

14. Anwendung nach einem der vorangehenden Ansprüche, wobei der Säuger ein Mensch ist.

15. Anwendung nach einem der vorangehenden Ansprüche, wobei jeder Partikel ein Metallpartikel von 0,5 bis 5 $\mu$m Größe ist

16. Anwendung nach Anspruch 15, wobei der Partikel ein amorpher Goldpartikel von 0,5 bis 3 $\mu$m Größe ist.

17. Anwendung eines immunogenen Peptids oder Proteins in der Herstellung eines aus Partikeln bestehenden Medikaments für die Impfung von Menschen, worin:

   - das immunogene Peptid oder Protein auf biologisch inerte Partikel aufgeschichtet wird,
   - die Impfung durch Akzelerierung der beschichteten Partikel in Ziel-Hautzellen eines Menschen hinein erfolgt, und
   - die biologisch inerten Partikel eine ausreichende Größe und Dichte aufweisen, um direkt in die Zielzellen beförderbar zu sein.

**Revendications**

1. Utilisation d'un immunogène dans la fabrication d'un médicament particulaire pour accélération dans une cellule cible d'un sujet mammifère en une quantité suffisante pour induire chez le mammifère une réponse immunitaire spécifique audit immunogène, dans laquelle l'immunogène est déposé sous la forme d'un revêtement sur une particule biologiquement inerte ayant une densité suffisante pour être délivrée directement dans la cellule cible.

2.  Utilisation selon la revendication 1, dans laquelle la réponse immunitaire est **caractérisée par** une réponse cellulaire spécifique dudit immunogène.

3.  Utilisation selon la revendication 1, dans laquelle la réponse immunitaire est **caractérisée par** une réponse anticorps spécifique dudit immunogène.

4.  Utilisation selon la revendication 1, dans laquelle la réponse immunitaire est **caractérisée par** à la fois une réponse cellulaire et une réponse anticorps spécifiques dudit immunogène.

5.  Utilisation selon l'une quelconque des revendications précédentes, dans laquelle la particule revêtue est accélérée dans la cellule par la force générée par une décharge gazeuse.

6.  Utilisation selon l'une quelconque des revendications 1 à 4, dans laquelle la particule revêtue est accélérée dans la cellule par la force générée par une décharge électrique.

7.  Utilisation selon l'une quelconque des revendications précédentes, dans laquelle l'immunogène est chimiquement lié à la particule.

8.  Utilisation selon l'une quelconque des revendications précédentes, dans laquelle l'immunogène est un peptide.

9.  Utilisation selon la revendication 8, dans laquelle le peptide a moins de 15 résidus d'acides aminés.

10. Utilisation selon l'une quelconque des revendications 1 à 7, dans laquelle l'immunogène est un polypeptide.

11. Utilisation selon l'une quelconque des revendications 1 à 7, dans laquelle l'immunogène est un glycopeptide.

12. Utilisation selon l'une quelconque des revendications 1 à 7, dans laquelle l'immunogène est un virus inactivé entier.

13. Utilisation selon l'une quelconque des revendications précédentes, dans laquelle la cellule cible est une cellule cutanée.

14. Utilisation selon l'une quelconque des revendications précédentes, dans laquelle le sujet mammifère est un humain.

15. Utilisation selon l'une quelconque des revendications précédentes, dans laquelle chaque particule est une particule métallique de 0,5 à 5 $\mu$m de taille.

16. Utilisation selon la revendication 15, dans laquelle la particule est une particule d'or amorphe de 0,5 à 3 $\mu$m de taille.

17. Utilisation d'un peptide ou d'une protéine immunogène dans la fabrication d'un médicament particulaire pour la vaccination humaine, dans laquelle :

    -   le peptide ou la protéine immunogène est déposé sous la forme d'un revêtement sur des particules biologiquement inertes,
    -   ladite vaccination est effectuée en accélérant les particules revêtues dans des cellules cutanées cibles d'un humain, et
    -   les particules biologiquement inertes ont une taille et une densité suffisantes pour être délivrées directement dans les cellules cibles.

# FIG. 1

**Weights of Surviving Mice**

FIG 2

**Symptoms After Flu Virus Challenge**

FIG 3

**Survival of Lethal Flu Challenge**

FIG 4

SEQUENCE LISTING

(1) GENERAL INFORMATION:

(i) APPLICANT: Pohlman, Edward C.
Sheehy, Michael J.
Barton, Kenneth A.

(ii) TITLE OF INVENTION: Method for Inducing Immune Response

(iii) NUMBER OF SEQUENCES: 1

(iv) CORRESPONDENCE ADDRESS:

    (A) ADDRESSEE: Quarles and Brady
    (B) STREET: PO Box 2113
    (C) CITY: Madison
    (D) STATE: WI
    (E) COUNTRY: USA
    (F) ZIP: 53701

(v) COMPUTER READABLE FORM:

    (A) MEDIUM TYPE: Floppy disk
    (B) COMPUTER: IBM PC compatible
    (C) OPERATING SYSTEM: PC-DOS/MS-DOS
    (D) SOFTWARE: PatentIn Release #1.0, Version #1.25

(vi) CURRENT APPLICATION DATA:

    (A) APPLICATION NUMBER:
    (B) FILING DATE:
    (C) CLASSIFICATION:

(viii) ATTORNEY/AGENT INFORMATION:

    (A) NAME: Seay, Nicholas J
    (B) REGISTRATION NUMBER: 27,386
    (C) REFERENCE/DOCKET NUMBER: 11-229-9105-5

(ix) TELECOMMUNICATION INFORMATION:

    (A) TELEPHONE: 608-251-5000
    (B) TELEFAX: 608-251-9166

(2) INFORMATION FOR SEQ ID NO:1:

(i) SEQUENCE CHARACTERISTICS:

    (A) LENGTH: 9 amino acids
    (B) TYPE: amino acid
    (D) TOPOLOGY: linear

(ii) MOLECULE TYPE: peptide

(v) FRAGMENT TYPE: internal

(vi) ORIGINAL SOURCE:

    (A) ORGANISM: Influenza virus

(xi) SEQUENCE DESCRIPTION: SEQ ID NO:1:

```
Thr Tyr Gln Arg Thr Arg Ala Leu Val
1                   5
```